# EUROPÄISCHE PATENTSCHRIFT

(11) **EP 4 230 744 B1**
(45) Veröffentlichungstag und Bekanntmachung des Hinweises auf die Patenterteilung: **23.07.2025**
(21) Anmeldenummer: 22157431.2
(22) Anmeldetag: 18.02.2022
(51) Int. Cl.: C12Q 1/22, B01L 9/00, C12M 1/22, G01N 1/22, G01N 35/00

(54) **VORRICHTUNG UND VERFAHREN ZUM MESSEN DER VERKEIMUNG EINES KONTROLLIERTEN, NAHEZU KEIMFREIEN RAUMES, INSBESONDERE EINES REINRAUMES, MITTELS EINER IN EINER PETRISCHALE BEFINDLICHEN, KEIME DETEKTIERENDEN NÄHRLÖSUNG**
DEVICE AND METHOD FOR MEASURING THE MICROBIAL CONTAMINATION OF A CONTROLLED, VIRTUALLY GERM-FREE SPACE, IN PARTICULAR A CLEAN ROOM, BY MEANS OF A NUTRIENT SOLUTION IN A PETRI DISH
DISPOSITIF ET PROCÉDÉ DE MESURE DE LA PROLIFÉRATION DE GERMES DANS UN ESPACE QUASI-STÉRILE CONTRÔLÉ, EN PARTICULIER D'UNE SALLE BLANCHE, AU MOYEN D'UNE SOLUTION NUTRITIONNELLE DÉTECTRICE DE GERMES SE TROUVANT DANS UNE BOÎTE DE PÉTRI

(43) Veröffentlichungstag der Anmeldung: 23.08.2023
(73) Patentinhaber: ACCURRO GmbH, 35041 Marburg (DE)
(72) Erfinder: BATTENBERG, Ralf, 35037 Marburg (DE); WAGNER, Alexander, 35102 Lohra (DE)
(74) Vertreter: Walther Bayer Faber Patentanwälte PartGmbB

(56) Entgegenhaltungen:
- WO-A1-2015/013374
- WO-A1-2021/130544
- DE-A1- 102020 130 016
- DE-U1- 202018 003 348
- GB-A- 2 584 787

## Beschreibung

Die vorliegende Erfindung betrifft eine Vorrichtung zum Messen der Verkeimung eines kontrollierten Raumes, insbesondere eines Reinraumes, mittels einer in einer Petrischale befindlichen, Keime detektierenden Nährlösung gemäß dem Oberbegriff des Anspruches 1 und ein entsprechendes Verfahren gemäß dem Oberbegriff des Anspruches 14.

Die Herstellung von Medikamenten soll in vielen Fällen in einer sterilen und/oder nahezu keimfreien Umgebung erfolgen, weshalb derartige Medikamente in auf Keime kontrollierten Räumen hergestellt, verarbeitet und/oder verpackt werden. In diesen kontrollierten Räumen wird die Keimbelastung durch geeignete Maßnahmen entsprechend den Anforderungen reduziert. Bei kontrollierten Räumen mit einer extrem niedrigen Keimbelastung spricht mach auch von Reinräumen.

Während der Herstellung, der Verarbeitung und/oder der Verpackung der Medikamente muss aus Gründen der Qualitätssicherung nachgewiesen werden, dass die zulässige Keimbelastung in dem kontrollierten Raum nicht überschritten wurde. Hierzu wird in dem kontrollierten Raum für eine bestimmte Zeit, zum Beispiel für 20 Minuten, eine Keime detektierende Nährlosung ausgesetzt. Dies geschieht dadurch, dass vor Beginn der Verarbeitung des Medikamentes in dem kontrollierten Raum eine Anzahl von eine geeignete Nährlösung tragenden Petrischalen platziert werden. Es versteht sich, dass die Petrischalen zu diesem Zeitpunkt noch keimdicht verschlossen sind.

Nachdem der kontrollierte Raum geschlossen wurde und nachdem die Keimbelastung im kontrollierten Raum auf das gewünschte Maß reduziert wurde und ggf. nachdem mit der Verarbeitung des Medikamentes begonnen wurde, wird eine erste Petrischale geöffnet. Dies geschieht dadurch, dass ein Bediener vorzugsweise über einen in einer Wand des kontrollierten Raumes vorhandenen, keimdichten Handschuh, den Deckel der ersten Petrischale ergreift, abhebt und beiseite legt, so dass die im Boden der Petrischale befindliche Nährlösung nun eventuell im abgeschlossenen Raum befindliche Keime aufnehmen kann. Nach einer vorgegebenen Zeit, zum Beispiel 20 Minuten, wird der Bediener, wieder unter Nutzung des keimfreien Handschuhs, den Deckel der Petrischale aufnehmen und auf den die Nährlösung tragenden Boden der Petrischale aufsetzen und damit die Nährlösung keimdicht abschließen. Zu gegebener Zeit wird auf die gleiche Art und Weise eine zweite Petrischale geöffnet und nach der vorgegebenen Zeit wieder geschlossen. Dabei werden entsprechend den durch die Qualitätsstandards vorgegebenen Bedingungen entsprechend viele Petrischalen auf die oben beschriebene Weise geöffnet und wieder geschlossen.

Nachdem die Herstellung, Verarbeitung und/oder Verpackung der Medikamente abgeschlossen ist, werden die wieder keimdicht verschlossen Petrischalen aus dem nunmehr offenen, kontrollierten Raum herausgeholt und in ein Labor verbracht. Dort werden die sich in der Nährlösung befindlichen Keime ausgewertet.

Es versteht sich, dass es für den Bediener sehr schwierig ist, unter Verwendung des keimfeien Handschuhs den vergleichsweise kleinen Deckel der Petrischale zu ergreifen, um die Petrischale zu öffnen und ordnungsgemäß wieder zu schließen.

Zur Behebung dieses Nachteiles ist aus der DE 20 2018 003 348 U1 ein elektrisch betriebener Petrischalenbetätiger zum zuverlässigen Öffnen und Schließen der Petrischale bekannt, der von außerhalb des kontrollierten Raumes bedient werden kann. Hierdurch entfällt das schwierige und aufwendige manuelle Öffnen und Schließen der Petrischale.

Für den Fall, dass die tatsächliche Keimbelastung für einen längeren Zeitraum festgestellt werden soll oder für den Fall, dass die Keimbelastung für verschiedene Chargen unabhängig voneinander festgestellt werden soll, müssen mehrere Petrischalenbetätiger installiert werden, was verhältnismäßig aufwendig ist.

Weitere Vorrichtungen, mit denen Petrischalen gehandhabt werden können, sind in der WO 2021/130544 A1, der GB 2 584 787 A und der WO 2015/013374 A1 offenbart.

Davon ausgehend liegt der vorliegenden Erfindung die Aufgabe zu Grunde, eine Vorrichtung und ein Verfahren der eingangs genannten Art zu schaffen, mit dem die Keimbelastung für verschiedene zeitliche Abschnitte kostengünstig realisiert werden kann.

Als technische Lösung dieser Aufgabe wird erfindungsgemäß eine Vorrichtung mit den Merkmalen des Anspruches 1 und ein Verfahren mit den Merkmalen des Anspruches 14 vorgeschlagen. Vorteilhafte Weiterbildungen dieser Vorrichtung und dieses Verfahrens sind den jeweiligen Unteransprüchen zu entnehmen.

Eine nach dieser technischen Lehre ausgebildete Vorrichtung und ein nach dieser technischen Lehre ausgeführtes Verfahren haben den Vorteil, dass im Petrischalenspeicher eine große Anzahl von Petrischalen bereitgestellt werden können, wobei jede einzelne Petrischale von der Transportvorrichtung dem Petrischalenbetätiger zugeführt und nach erfolgter Messung wieder in den Petrischalenspeicher zurückgeführt werden kann, so dass eine Dokumentation der Verkeimung im kontrollierten Raum über einen längeren Zeitraum und/oder für verschiedene Chargen automatisiert und damit kostengünstig durchgeführt werden kann.

Dabei hat es sich als vorteilhaft erwiesen, die Transportvorrichtung mit einem schwenkbar gehaltenen Schwenkturm samt Petrischalenaufnahme oder einer Führungsbahn mit Schubhebel auszustatten so dass die Petrischale automatisiert vom Petrischalenspeicher zum Petrischalenbetätiger gebracht und nach erfolgter Messung wieder zurückgebracht werden kann.

Den Petrischalenspeicher erfindungsgemäß mit einem vertikal ausgerichteten Hubturm auszustatten hat den Vorteil, dass so eine Anzahl von Petrischalen platzsparend im kontrollierten Raum untergebracht werden können. Ein weiterer Vorteil besteht darin, dass der Hubturm vertikal verschiebbar ist, so dass die einzusetzende Petrischale in einfacher Weise und automatisiert auf das Niveau der Petrischalenaufnahme oder die Führungsbahn der Transportvorrichtung gebracht werden kann.

Die Petrischalenablagen abnehmbar am Hubturm anzubringen hat den Vorteil, dass die Petrischalenablage in einfacher Weise abgebaut und aus dem kontrollierten Raum herausgebracht werden kann, so dass die Entkeimung und Sterilisation an einer dafür vorbestimmten Stelle sehr viel gründlicher und kostengünstiger durchgeführt werden kann. Ein weiterer Vorteil besteht darin, dass die Petrischalenablage nach der Entkeimung und Sterilisation auch gleich mit frisch gefüllten Petrischalen bestückt werden kann. Anschließend kann die Petrischalenablage samt der darin gehaltenen Petrischalen steril verpackt und zu gegebener Zeit wieder in den kontrollierten Raum zur weiteren Verwendung verbracht werden.

Die Petrischalen übereinander fluchtend anzubringen hat den Vorteil, dass so mittels einer vertikalen Verstellung des Hubturmes die Petrischalenablage in einfacher Weise auf das Niveau der Transportvorrichtung gebracht wird

Die Ausbildung einer Vertiefung in der Petrischalenablage hat den Vorteil, dass so die Petrischale zuverlässig gehalten wird und ein Herausrutschen aus der Petrischalenablage vermieden wird.

Die Ausstattung der Transportvorrichtung mit einem schwenkbar gehaltenen Schwenkturm und einer am Schwenkturm radial abstehend gehaltenen Petrischalenaufnahme hat den Vorteil, dass so die Petrischale schnell und in einfacher Weise und vor allem automatisiert vom Petrischalenspeicher zum Petrischalenbetätiger überführt werden kann. Ein weiterer Vorteil besteht darin, dass hierdurch nur sehr wenig Platz im Reinraum benötigt wird, insbesondere wenn diese Überführung mit einem Schwenkbereich von 90 Grad oder 180 Grad möglich ist.

Die Ausbildung von Haltefingern an der Petrischalenaufnahme hat den Vorteil, dass somit die Petrischale vollständige aufgegriffen werden kann, wodurch ein zuverlässiger Transport möglich wird und dass gleichzeitig eine Interaktion mit der Petrischalenablage möglich ist, wodurch die Petrischale ohne mechanisch verschoben werden zu müssen aus der Petrischalenablage herausgebracht werden kann. Analoges gilt bei der Rückführung der Petrischale vom Petrischalenbetätiger zur Petrischalenablage, denn auch hier ist eine Überführung möglich, ohne die Petrischale mechanisch verschieben zu müssen.

Die Ausbildung einer vertikal ausgerichteten Anlageschulter in der Petrischalenaufnahme bewirkt, dass durch diese formschlüssige Barriere ein Herausfallen der Petrischale aus der Petrischalenaufnahme während des Transportes der Petrischale zuverlässig vermieden wird, was bei einem automatisierten Transport sehr vorteilhaft ist.

In einer weiteren, bevorzugten Ausführungsform umfasst die Transportvorrichtung eine Führungsbahn, vorzugsweise mit einem einen Auflagesteg aufweisenden Außenring und einem einen Auflagesteg aufweisenden Innenring, wobei die Petrischale auf den beiden Auflagestegen aufliegend mittels eines Schubhebels von dem Petrischalenspeicher zum Petrischalenbetätiger verschoben werden. Dies hat den Vorteil, dass hierdurch die Petrischale zuverlässig und automatisiert vom Petrischalenspeicher zum Petrischalenbetätiger und zurückgebracht werden kann, insbesondere wenn ein am Außenring und/oder am Innenring vorgesehener Anlagesteg die Petrischale am Verlassen der Auflagestege hindert.

Weitere Vorteile der erfindungsgemäßen Vorrichtung und des erfindungsgemäßen Verfahrens ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen innerhalb des Schutzumfanges miteinander verwendet werden. Die erwähnten Ausführungsformen sind nicht als abschließende Aufzählung zu verstehen, sondern haben vielmehr beispielhaften Charakter. Es zeigen:
- Fig. 1: eine perspektivische Frontansicht einer ersten Ausführungsform der erfindungsgemäßen Vorrichtung;
- Fig. 2: eine Seitenansicht der Vorrichtung gemäß Fig. 1;
- Fig. 3: eine Draufsicht der Vorrichtung gemäß Fig. 1;
- Fig. 3a: eine Detailvergrößerung der Vorrichtung gemäß Fig. 1, entsprechend Linie IIIa in Fig. 3;
- Fig. 4: eine Detailvergrößerung der Petrischalenablage der Vorrichtung gemäß Fig. 1, entsprechend Linie IV in Fig. 1;
- Fig. 5: eine Detailvergrößerung des Petrischalenbetätigers der Vorrichtung gemäß Fig. 1, entsprechend Linie V in Fig. 2;
- Fig. 6: eine perspektivische Frontansicht der mit Petrischalen bestückten Vorrichtung gemäß Fig. 1 zu einem ersten Zeitpunkt (Ausgangsstellung);
- Fig. 7: eine perspektivische Frontansicht der Vorrichtung gemäß Fig. 6 zu einem zweiten Zeitpunkt;
- Fig. 8: eine perspektivische Frontansicht der Vorrichtung gemäß Fig. 6 zu einem dritten Zeitpunkt;
- Fig. 9: eine perspektivische Frontansicht der Vorrichtung gemäß Fig. 6 zu einem vierten Zeitpunkt;
- Fig. 10: eine perspektivische Frontansicht der Vorrichtung gemäß Fig. 6 zu einem fünften Zeitpunkt;
- Fig. 11: eine perspektivische Frontansicht der Vorrichtung gemäß Fig. 6 zu einem sechsten Zeitpunkt;
- Fig. 12: eine perspektivische Frontansicht der Vorrichtung gemäß Fig. 6 zu einem siebten Zeitpunkt;
- Fig. 13: eine perspektivische Ansicht einer mit Petrischalen bestückten zweiten Ausführungsform der erfindungsgemäßen Vorrichtung zu einem ersten Zeitpunkt (Ausgangsstellung);
- Fig. 14: eine perspektivische Ansicht der Vorrichtung gemäß Fig. 13 zu einem zweiten Zeitpunkt;
- Fig. 15: eine perspektivische Ansicht der Vorrichtung gemäß Fig. 13 zu einem dritten Zeitpunkt;
- Fig. 16: eine perspektivische Frontansicht der Vorrichtung gemäß Fig. 13 zu einem vierten Zeitpunkt;
- Fig. 17: eine perspektivische Frontansicht der Vorrichtung gemäß Fig. 13 zu einem fünften Zeitpunkt;
- Fig. 18: eine perspektivische Frontansicht der Vorrichtung gemäß Fig. 13 zu einem sechsten Zeitpunkt.
- Fig. 19: eine perspektivische Ansicht eines Petrischalenspeichers einer dritten Ausführungsform der erfindungsgemäßen Vorrichtung;

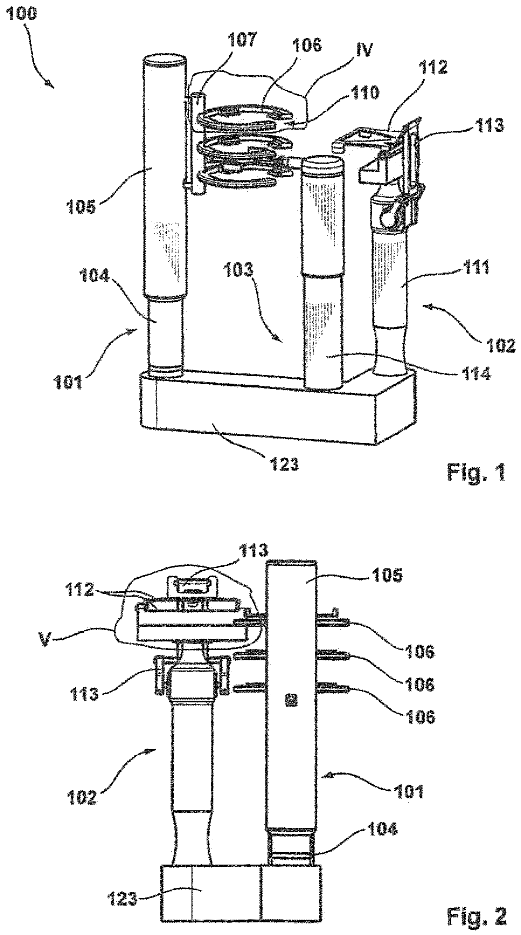

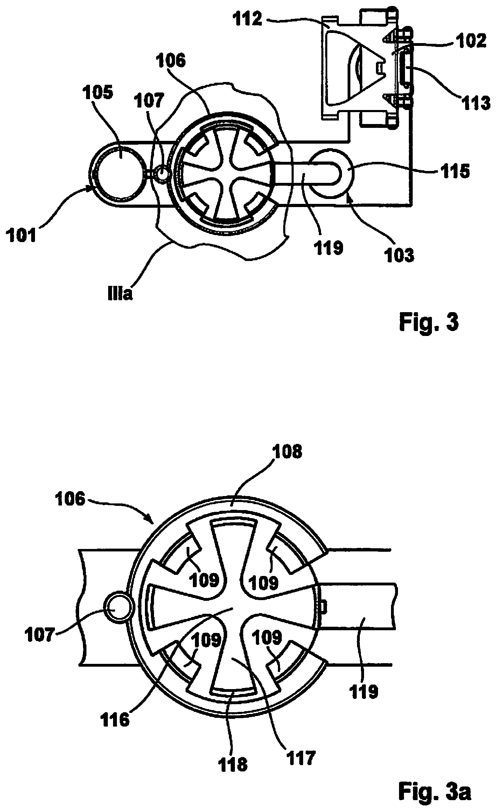

In den Figuren 1 bis 3 ist eine erste Ausführungsform einer erfindungsgemäßen Vorrichtung zum Messen der Verkeimung eines kontrollierten, nahezu keimfreien Raumes dargestellt, wobei die Verkeimung mittels einer hier nicht dargestellten, in einer Petrischale befindlichen, Keime detektierenden Nährlösung durchgeführt wird. Diese Vorrichtung zum Messen der Verkeimung 100 umfasst einen Petrischalenspeicher 101 zur Aufbewahrung von drei geschlossenen, hier nicht dargestellten Petrischalen, einen Petrischalenbetätiger 102 zum Öffnen und Schließen einer solchen Petrischale und eine Transportvorrichtung 103 zum Transportieren der Petrischale vom Petrischalenspeicher 101 zum Petrischalenbetätiger 102 und zurück.

Der Petrischalenspeicher 101 umfasst einen im Wesentlichen zylindrischen Speichersockel 104, einen hohlzylindrischen, auf dem Speichersockel 104 gehaltenen Hubturm 105 und in dieser Ausführungsform drei am Hubturm 105 angebrachte Petrischalenablagen 106, auf denen die hier nicht dargestellten Petrischalen im geschlossenen Zustand abgelegt werden können. In vielen Anwendungen sind drei Petrischalen zum Vermessen der Verkeimung im Raum ausreichend. Sollten einmal mehr als drei Petrischalen benötigt werden, so können in einer anderen Ausführungsform auch mehr als drei, vorzugsweise acht oder auch zwölf, Petrischalenablagen am Hubturm angebracht sein.

Im Speichersockel 104 ist eine Verstellvorrichtung untergebracht, die auf den Hubturm 105 wirkt und diesen vertikal nach oben oder unten bewegen kann. Am Hubturm 105 ist eine Haltestange 107 angebracht, an der die drei Petrischalenablagen 106 beabstandet voneinander gehalten sind. Dabei sind die Petrischalenablagen 106 in vertikaler Richtung fluchtend miteinander angeordnet, sodass die hier aufzunehmenden Petrischalen zwar beabstandet voneinander aber ansonsten direkt übereinander angeordnet sind.

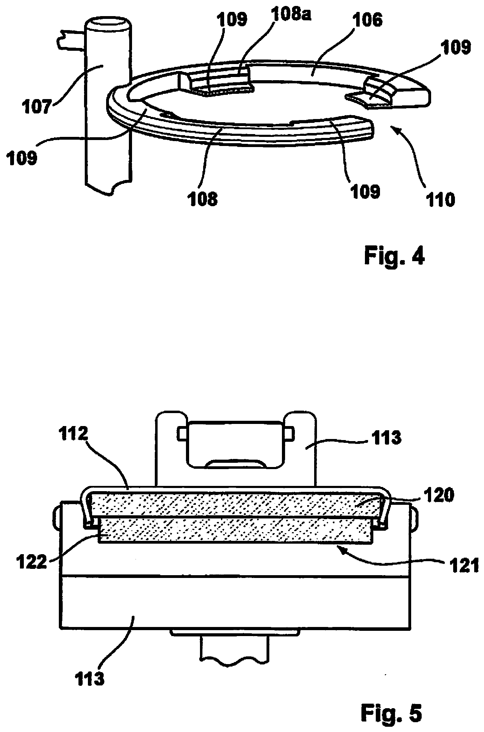

In Figur 4 ist eine solche Petrischalenablage 106 vergrößert dargestellt. Diese Petrischalenablage 106 umfasst einen Ablagering 108, an den vier nach innen ausgerichtete Anschläge 108a äquidistant angebracht sind. An jedem der Anschläge 108a sind an deren unteren Rand Ablagezungen 109 angebracht, sodass hier eine Vertiefung zur Aufnahme der Petrischale entsteht. Dabei kommt die Petrischale auf einer Oberseite der Ablagezunge 109 zur Ablage, während die radial nach innen vorstehenden Anschläge 108a einen formschlüssigen Halt der Petrischale in der Petrischalenablage 106 bilden. Es versteht sich, dass die Ablagezunge 109 und der Anschlag auf die Größe der darin abzulegenden Petrischale abgestimmt ist, so dass die Petrischale in der Petrischalenablage 106 Platz findet, vorzugsweise formschlüssig darin gehalten ist. Darüber hinaus besitzt der Ablagering 108 eine zwischen zwei benachbarten Ablagezungen 109 ausgebildete Öffnung 110, die genau gegenüber der Haltestange 107 angeordnet ist. Diese Öffnung 110 ermöglicht es der Transportvorrichtung 103 die Petrischale aus der Petrischalenablage 106 herauszubewegen, wie weiter unten ausführlicher beschrieben ist.

Wie aus den Figuren 1 bis 3 ersichtlich ist, umfasst der Petrischalenbetätiger 102 einen auf einem Ständer 111 gehaltenen Deckelgreifer 112 und einen am Ständer 111 angebrachten Mechanismus 113 zum Anheben und Absenken des Deckelgreifers 112, wobei der Mechanismus 113 den Deckelgreifer 112 in einer Kreisbogenbahn bewegt.

Wie ebenfalls aus den Figuren 1 bis 3 ersichtlich ist, umfasst die Transportvorrichtung 103 einen Transportsockel 114 und einen schwenkbar daran gehaltenen Schwenkturm 115, an dem eine radial abstehende und im Wesentlichen horizontal ausgerichtete Petrischalenaufnahme 116 gehalten ist. Im Transportsockel 114 ist ein hier nicht näher dargestellter Schwenkmechanismus untergebracht, der den Schwenkturm 115, ausgehend von seiner Ausgangsposition, um 90 Grad im Uhrzeigersinn verschwenken kann und der den Schwenkturm 115 ebenfalls wieder in seine Ausgangsposition zurückschwenken kann. In einer anderen Ausführungsform ist die Petrischalenaufnahme um 180 Grad oder bis zu 360 Grad schwenkbar.

Am Schwenkturm 115 ist eine Petrischalenaufnahme 116 vorgesehen, die vier äquidistant angeordnete Haltefinger 117 aufweist, wobei die Petrischalenaufnahme 116 und die Petrischalenablage 106 derart ausgestaltet sind, dass die Haltefinger 117 zwischen benachbarte Anschläge 108a bzw. Ablagezungen 109 reichen, ohne den Ablagering 108 zu berühren. Am äußeren Ende eines jeden Haltefingers 117 ist eine nach oben ausgerichtete Anschlagschulter 118 ausgebildet, an der die in der Petrischalenaufnahme 116 aufzunehmende Petrischale zum Anschlag kommen kann. Dabei ist die zur Petrischale ausgerichtete Anschlagfläche der Anschlagschulter 118 fluchtend mit der Anschlagfläche des Anschlages 108a des Ablagerings 108 ausgebildet, sodass die Petrischale sowohl passgenau in die Petrischalenablage 106, als auch passgenau in die Petrischalenaufnahme 116 hineinpasst. Ein Haltefinger 117 ist im Bereich der Öffnung 110 des Ablagerings 108 mit einen am Schwenkturm 115 angebrachten Haltesteg 119 verbunden.

In Figur 5 ist ein Teil des Petrischalenbetätigers 102 vergrößert dargestellt, wobei im Deckelgreifer 112 ein Deckel einer Petrischale 121 gehalten ist. Der ebenfalls zur Petrischale 121 gehörende Boden 122 befindet sich dabei in der Petrischalenaufnahme 116, die hier aber aus Gründen der besseren Sichtbarkeit nicht dargestellt ist. Der Deckelgreifer 112 umgreift den Deckel 120 formschlüssig, sodass der Deckel 120 beim Betätigen des Deckelgreifers 112 mit nach oben verschwenkt wird und sich somit die Petrischale 121 öffnet, sodass die im Raum befindlichen Keime an die in der Petrischale befindliche Nährlösung gelangen können.

In der in den Figuren 1 bis 5 dargestellten ersten Ausführungsform sind sowohl der Petrischalenspeicher 101, als auch der Petrischalenbetätiger 102 und die Transportvorrichtung 103 auf einer gemeinsamen Plattform 123 montiert, wobei der Petrischalenbetätiger 102 gegenüber dem Petrischalenspeicher 101 derart angeordnet ist, dass die am Schwenkturm 115 befestigte Petrischalenaufnahme 116, die im Petrischalenspeicher 101 befindliche Petrischale durch ein Verschwenken um 90 Grad in den Deckelgreifer 112 des Petrischalenbetätigers 102 überführt. Der genaue Ablauf dieses Vorgangs ist nachfolgend wie folgt beschrieben:

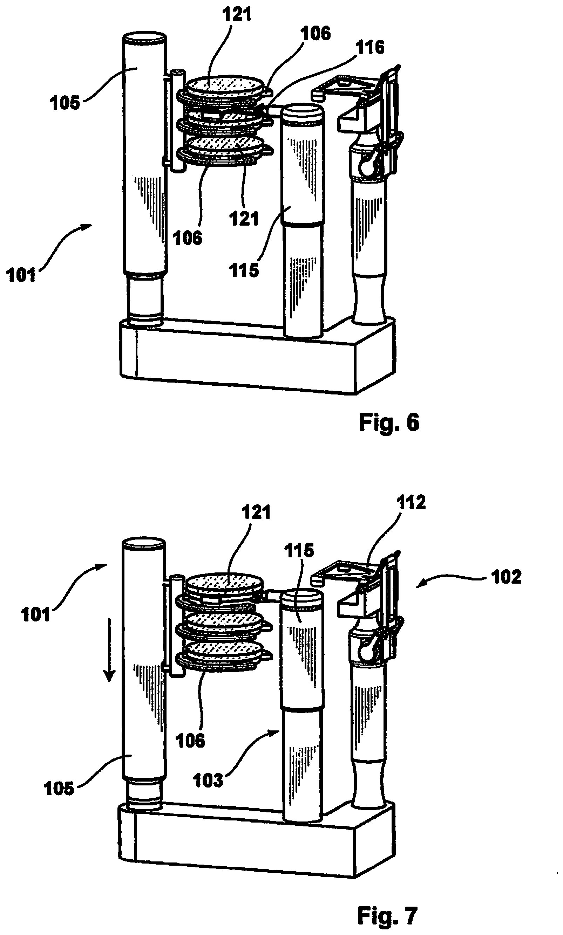

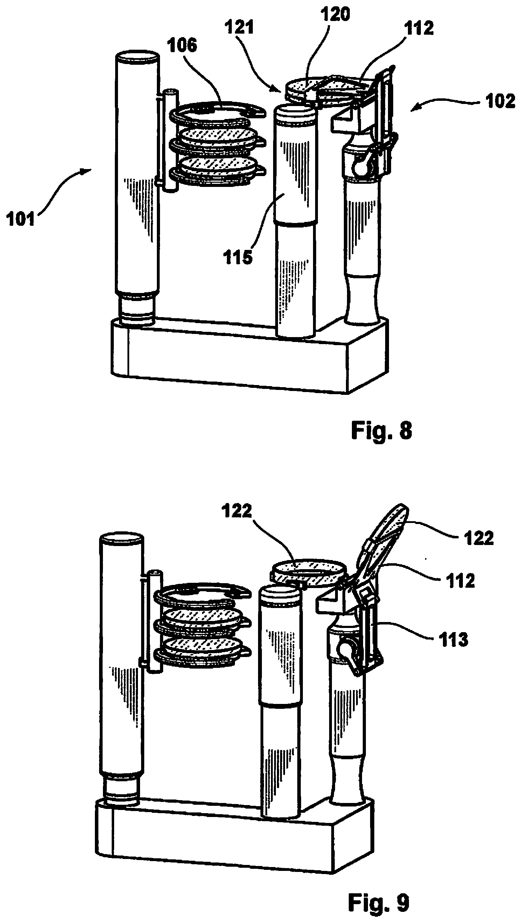

In den Figuren 6 bis 12 ist der Ablauf einer Messung der Verkeimung eines kontrollierten, nahezu keimfreien Raumes mittels einer in einer Petrischale befindlichen, Keime detektierenden Nährlösung dargestellt, welche mit der Vorrichtung gemäß den Figuren 1 bis 5 durchgeführt wird. Wie aus Figur 6 ersichtlich, befinden sich im Petrischalenspeicher 101 drei Petrischalen 121 in den jeweiligen Petrischalenablagen 106 des Hubturmes 105. Die Petrischalenaufnahme 116 des Schwenkturmes 115 der Transportvorrichtung 103 ist bereits in den Zwischenraum unterhalb der obersten Petrischalen-ablage 106 und oberhalb der mittleren Petrischalenablage 106 verbracht. Wie aus Fig. 7 ersichtlich wird im nächsten Schritt der Hubturm 105 so weit abgesenkt, dass die in der obersten Petrischalenablage 106 befindliche Petrischale 121 auf die Petrischalenaufnahme 116 der Transportvorrichtung 103 gelangt und dass sich die Petrischalenablage 106 unterhalb der Petrischalenaufnahme 116 befindet. Nun wird der Schwenkturm 115 um 90 Grad im Uhrzeigersinn verschwenkt, so dass die auf der Petrischalen-aufnahme 116 befindliche Petrischale 121 vom Petrischalenspeicher 101 zum Petrischalenbetätiger 102 verbracht wird, wie aus Fig. 8 ersichtlich ist. Dabei führt der Schwenkturm 115 den Deckel 120 der Petrischale 121 direkt in den Deckelgreifer 112, der den Deckel 120 formschlüssig umgreift. Anschließend wird der Deckelgreifer 112 vom Mechanismus 113 zum Anheben des Deckelgreifers 112 betätigt, so dass der Deckel 120 in einer Kreisbewegung vom Boden 122 der Petrischale 121 wegbewegt wird, um die in der Petrischale 121 befindliche Nährlösung den im kontrollierten Raum befindlichen Keimen auszusetzen, wie Fig. 9 zeigt. Dabei hält die Petrischalenaufnahme 116 der Transportvorrichtung 103 den Boden 122 der Petrischale 121 während der gesamten Messzeit in der Position gemäß Fig. 9.

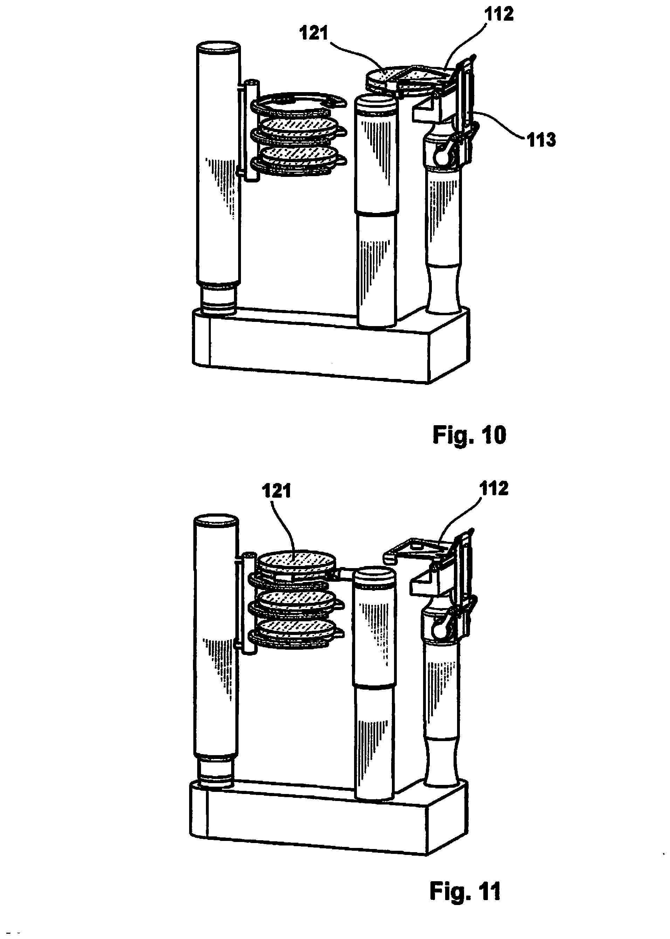

Nach Ablauf der vorgegebenen Zeitspanne zur Messung der Verkeimung im kontrollierten Raum wird der Deckel 120 durch den Mechanismus 113 zum Anheben des Deckelgreifers wieder auf den Boden 122 der Petrischale 121 aufgesetzt (Fig. 10) und anschließend bewegt der Schwenkturm 115 die Petrischalenaufnahme 116 entgegen des Uhrzeigersinns um 90 Grad wieder zurück in den Petrischalenspeicher 101, das heißt in eine Position oberhalb der obersten Petrischalenablage 106, wie aus Fig. 11 ersichtlich ist.

Anschließend wird der Hubturm 105 wieder nach oben bewegt und dabei wir die Petrischale 121 in der Petrischalenablage 106 abgelegt und die Petrischalenaufnahme 116 ist nun wieder leer. Danach wird die Petrischalenaufnahme 116 aus dem Bereich zwischen den Petrischalen 106 herausgeschwenkt (ohne Abbildung) und der Hubturm 105 wird soweit hochbewegt, dass sich die Petrischalenaufnahme 116 nun auf einer Höhe zwischen der mittleren und der unteren Petrischalenablage 106 befindet. Jetzt schwenkt der Schwenkturm 115 die Petrischalenaufnahme 115 entgegen dem Uhrzeigersinn zurück in den Hubturm 105 und die Nächste Petrischale 121 kann entnommen werden, analog zu dem Vorgang gemäß den Fig. 6 bis 12.

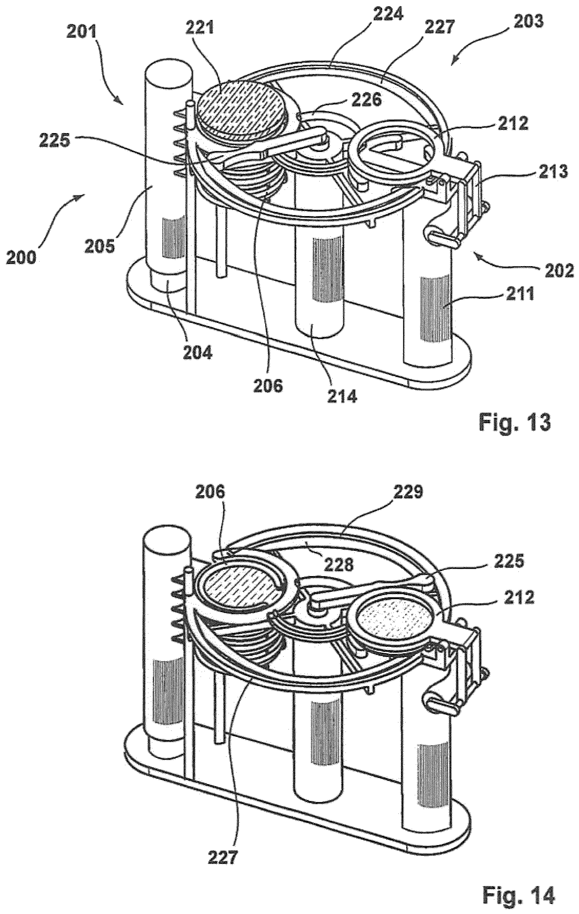

In den Figuren 13 bis 18 ist eine zweite Ausführungsform der erfindungsgemäßen Vorrichtung dargestellt. Diese zweite Ausführungsform umfasst einen Petrischalenspeicher 201 zur Aufbewahrung von vier eine Keime detektierende Nährlösung aufweisenden Petrischalen 221, einen Petrischalenbetätiger 202 zum Öffnen und Schließen der Petrischale 221 und eine Transportvorrichtung 203 zum Transportieren der Petrischale 221 vom Petrischalenspeicher 201 zum Petrischalenbetätiger 202 und zurück.

Der Petrischalenspeicher 201 umfasst einen im Wesentlichen zylindrischen Speichersockel 204, einen hohlzylindrischen, auf dem Speichersockel 204 gehaltenen Hubturm 205 und in dieser Ausführungsform vier am Hubturm 205 angebrachte Petrischalenablagen 206, auf denen die Petrischalen 221 im geschlossenen Zustand abgelegt sind.

Im Speichersockel 204 ist eine Verstellvorrichtung untergebracht, die auf den Hubturm 205 wirkt und diesen vertikal nach oben oder unten bewegen kann. Die vier Petrischalenablagen 206 sind beabstandet voneinander und in vertikaler Richtung fluchtend miteinander am Hubturm 205 angebracht, sodass die vier hier aufzunehmenden Petrischalen zwar beabstandet voneinander aber ansonsten direkt übereinander angeordnet sind. Die Petrischalenablage 206 ist als kreisrunder Teilring ausgebildet an dessen Innenseite eine Ablageschulter 230 zur Ablage der Petrischale 221 ausgebildet ist.

Der Petrischalenbetätiger 202 umfasst einen auf einem Ständer 211 gehaltenen Deckelgreifer 212 und einen am Ständer 211 angebrachten Mechanismus 213 zum Anheben und Absenken des Deckelgreifers 212, wobei der Mechanismus 213 den Deckelgreifer 212 in einer Kreisbogenbahn bewegt.

Die Transportvorrichtung 203 umfasst einen Transportsockel 214, eine am Transportsockel 214 angebrachte stationäre Führungsbahn 224 und einen drehbar am Transportsockel 214 gehaltenen Schubhebel 225, wobei der Schubhebel 225 um 360 Grad sowohl im als auch entgegen dem Uhrzeigersinn um den Transportsockel 214 drehbar ist. Angetrieben wird der Schubhebel 225 von einem im Transportsockel befindlichen Antrieb.

Die Führungsbahn 224 umfasst einen Innenring 226 und einen Außenring 227, wobei der Innenring 226 genauso wie der Außenring 227 einen im Wesentlichen horizontal ausgerichteten Auflagesteg 228 zur Auflage der Petrischale 221 und einen im Wesentlichen vertikal ausgerichteten Anlagesteg 229 aufweist, so dass die Petrischale 221 auf dem Auflagesteg 228 gleitend und vom Anschlagsteg 229 geführt vom Petrischalenspeicher 201 zum Petrischalenbetätiger 202 und zurück gelangt. Dabei wird die Petrischale 221 vom Schubhebel 225 entsprechend geschoben.

Der Deckelgreifer 212 umfasst einen Haltering 230 an dessen Unterseite zwei hier nicht dargestellte Vorsprünge angebracht sind, die den Deckel 220 der Petrischale klemmend halten. Hebt der Mechanismus 213 den Deckelgreifer 212 ab, so wird damit auch der klemmend zwischen den Vorsprüngen gehalten Deckel 220 abgehoben. Dementsprechend wird auch der Deckel 220 der Petrischale 221 wieder auf den Boden 222 der Petrischale 221 aufgesetzt, sobald der Deckelgreifer 212 abgelassen wird.

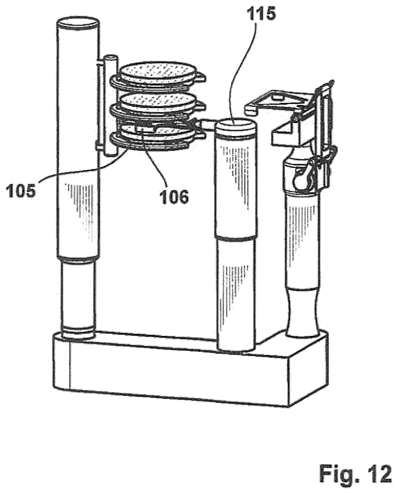

In den Figuren 13 bis 18 ist der Ablauf einer Messung der Verkeimung eines kontrollierten, nahezu keimfreien Raumes mittels einer in einer Petrischale befindlichen, Keime detektierenden Nährlösung dargestellt, welche mit der zweiten Ausführungsform gemäß den Figuren 13 bis 18 durchgeführt wird. Wie aus Figur 12 ersichtlich, befinden sich im Petrischalenspeicher 201 vier Petrischalen 221 in den jeweiligen Petrischalenablagen 206 des Hubturmes 205. Der Schubhebel 225 der Transportvorrichtung 203 befindet sich in seiner Ausgangsposition nahe der Petrischale 221 und der Hubturm 205 ist so hochgefahren, dass die oberste Petrischalenablage 206 mit dem Auflagesteg 228 der Führungsbahn 224 fluchtet. Zur Vorbereitung der Messung der Verkeimung schiebt nun der Schubhebel 225 die oberste Petrischale 221 aus der Petrischalenablage 206 heraus auf die Führungsbahn 224 und im Uhrzeigersinn weiter bis zum Petrischalenbetätiger 202, wobei der Schubhebel 225 den Deckel 220 der Petrischale 221 zwischen die Vorsprünge an der Unterseite des Deckelgreifers 212 drückt, so dass der Deckel 220 klemmend am Deckelgreifer 212 gehalten ist, wie dies in Fig. 14 dargestellt ist.

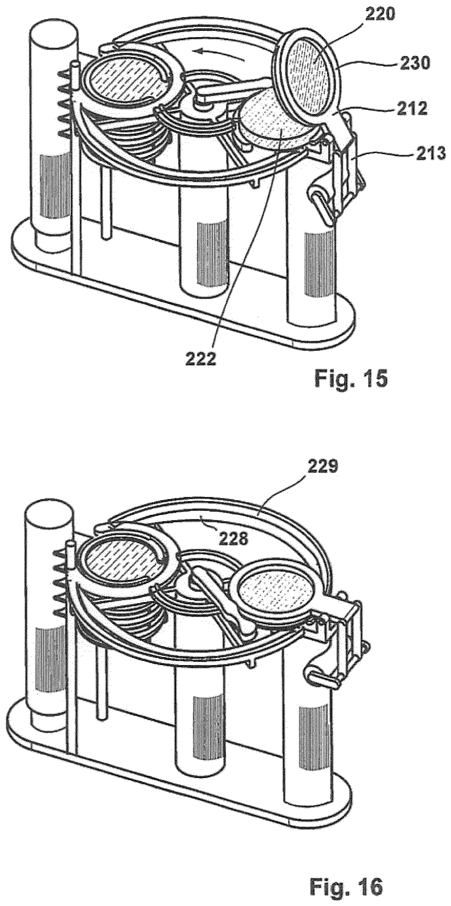

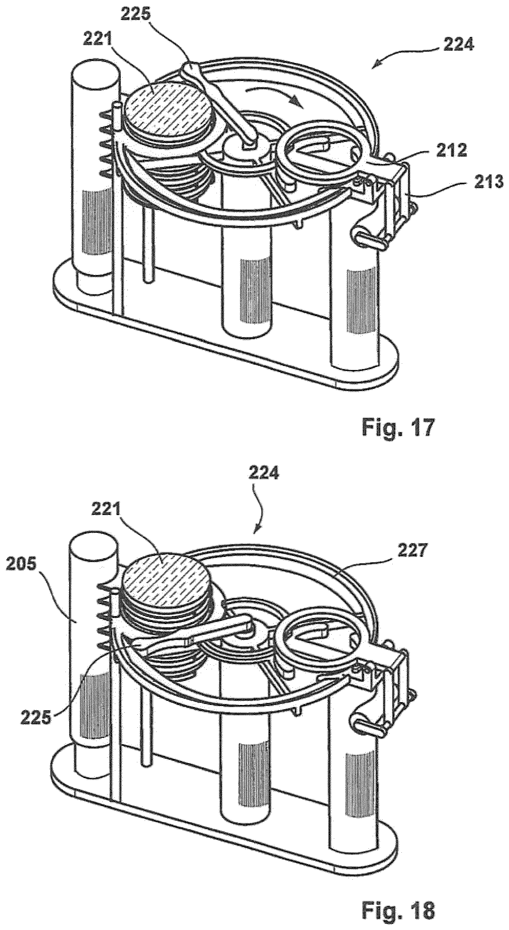

Sobald die Messung beginnen soll, wird der Mechanismus 213 zum Öffnen des Deckelgreifers 2112 betätigt und der Deckel 220 der Petrischale 221 wird abgehoben, so dass die im Boden 222 der Petrischale 221 befindliche Nährlösung in der Luft befindliche Keime aufnehmen und detektieren kann. Währenddessen wird der Schubhebel 225 entgegen dem Uhrzeigersinn auf die andere Seite der Petrischale 221 bewegt, wie Fig. 15 zeigt. Nach Abschluss der Messung, zum Beispiel nach 20 Minuten, schließt der Mechanismus 213 zum Betätigen des Deckelgreifers 212 die Petrischale 221 wieder, indem der Deckelgreifer 212 herunterbewegt wird und somit der Deckel 220 auf den Boden 222 der Petrischale 221 aufsetzt, entsprechend Fig. 16. Anschließend schiebt der Schubhebel 225 die verschlossene Petrischale 221 entgegen dem Uhrzeigersinn zurück in die Petrischalenablage 206 des Petrischalenspeichers 201 und bewegt sich dann im Uhrzeigersinn zurück zu seiner Ausgangsposition, wie Fig. 17 zeigt. Abschließend wird der Hubturm 205 so weit hochgefahren, dass die nächste Petrischale 221 auf die Höhe der Führungsbahn gelangt, wie Fig. 18 zeigt. Nun kann zu gegebener Zeit die zweite Petrischale 221 wie oben beschrieben zum Messen der Verkeimung eines kontrollierten Raumes eingesetzt werden.

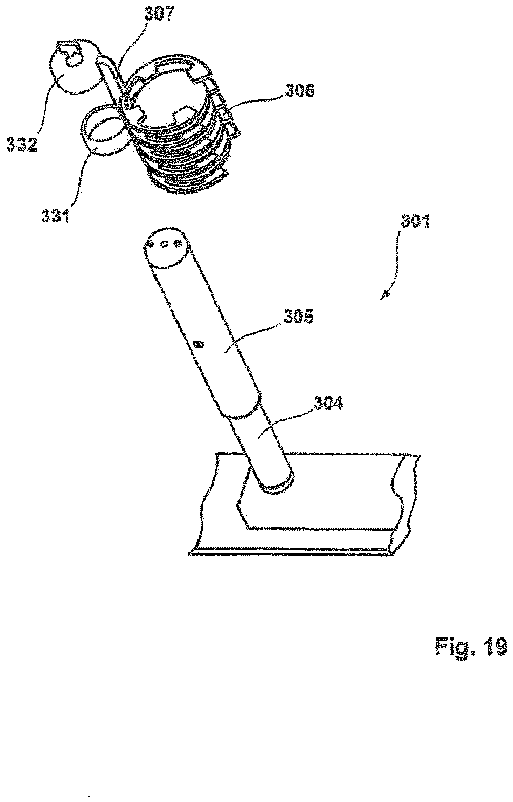

Fig. 19 zeigt einen Petrischalenspeicher 301 einer dritten Ausführungsform, der wie der Petrischalenspeicher 101 der ersten Ausführungsform einen im Wesentlichen zylindrischen Speichersockel 304, einen hohlzylindrischen, auf dem Speichersockel 304 gehaltenen Hubturm 305 und in dieser Ausführungsform fünf am Hubturm 305 angebrachte Petrischalenablagen 306 aufweist. Diese Petrischalenablagen 306 sind baugleich wie die Petrischalenablage der ersten Ausführungsform 106, ebenfalls an einer Haltestange 307 befestigt und ebenfalls beabstandet voneinander und übereinander fluchtend angeordnet. An dieser Haltestange 307 ist ein unterer, ringförmiger Kranz 331 und ein oberer, hutförmiger Halter 332 angebracht, wobei der Kranz 331 und der Hut 332 korrespondierend zum Hubturm 305 ausgebildet sind, sodass der Kranz 331 und der Halter 332 passgenau auf den Hubturm 305 aufgeschoben werden können, um die Petrischalenablagen 306 abnehmbar am Hubturm 305 zu halten.

## Patentansprüche

1. Vorrichtung zum Messen der Verkeimung eines kontrollierten, nahezu keimfreien Raumes, insbesondere eines Reinraumes, mittels einer in einer Petrischale (121, 221) befindlichen, Keime detektierenden Nährlösung, mit einem Petrischalenspeicher (101, 201) zur Aufnahme von mindestens zwei Petrischalen (121, 221), mit einem Petrischalenbetätiger (102, 202) zum Öffnen und Schließen einer Petrischale (121, 221) und mit einer Transportvorrichtung (103, 203) zum Transportieren einer einzelnen Petrischale (121, 221) vom Petrischalenspeicher (101, 201, 301) zum Petrischalenbetätiger (102, 202) und/oder zum Transportieren einer einzelnen Petrischale (121, 221) vom Petrischalenbetätiger (102, 202) zum Petrischalenspeicher (101, 201)
**dadurch gekennzeichnet,**
**dass** der Petrischalenspeicher (101, 201, 301) einen Speichersockel (104, 204, 304) und einen am Speichersockel (104, 204, 304) vertikal verstellbar gehaltenen Hubturm (105, 205, 305) umfasst, wobei am Hubturm (105, 205, 305) mindestens zwei Petrischalenablagen (106, 206, 306) vorgesehen sind.

2. Vorrichtung nach Anspruch 1,
**dadurch gekennzeichnet,**
**dass** der Hubturm (105, 205, 305) mittels einer Verstellvorrichtung vertikal verfahrbar ist, wobei die Verstellvorrichtung im Speichersockel (104, 205, 305) angeordnet ist.

3. Vorrichtung nach einem der Ansprüche 1 oder 32,
**dadurch gekennzeichnet,**
**dass** die Petrischalenablagen (306) abnehmbar am Hubturm (305) gehalten sind.

4. Vorrichtung nach wenigstens einem der Ansprüche 1 bis 3,
**dadurch gekennzeichnet,**
**dass** mindestens zwei Petrischalenablagen (106, 206, 306) fluchtend übereinander angeordnet sind.

5. Vorrichtung nach wenigstens einem der Ansprüche 1 bis 4,
**dadurch gekennzeichnet,**
**dass** die Petrischalenablage (106, 306) einen Ablagering (108) aufweist, an dem mindestens zwei, vorzugsweise vier, nach innen ausgerichtete Ablagezungen (109) ausgebildet sind, wobei die Ablagezungen (109) in einem unteren Bereich des Ablagerings(108) angeordnet sind und somit eine Vertiefung zur Aufnahme der Petrischale (121) ausbilden und insbesondere wobei im Ablagering (108) eine zur Transportvorrichtung (103) hin ausgerichtete Öffnung (110) ausgebildet ist.

6. Vorrichtung nach wenigstens einem der vorangehenden Ansprüche,
**dadurch gekennzeichnet,**
**dass** die Transportvorrichtung (103) einen Transportsockel (114) und einen am Transportsockel (114) schwenkbar gehaltenen Schwenkturm (115) umfasst, wobei am Schwenkturm (115) eine radial abstehende Petrischalenaufnahme (116) zur Aufnahme einer, vorzugsweise geschlossenen, Petrischale (121) ausgebildet ist.

7. Vorrichtung nach Anspruch 6,
**dadurch gekennzeichnet,**
**dass** der Schwenkturm (115) derart schwenkbar ausgebildet und angeordnet ist, dass die Petrischalenaufnahme (116) sowohl unter die Petrischalenablage (106) des Petrischalenspeichers (101), als auch unter den Petrischalenbetätiger (102) schwenkbar ist, insbesondere, dass der Schwenkturm (115) einen Schwenkbereich von 45 Grad bis 360 Grad, vorzugsweise von 80 Grad bis 210 Grad, insbesondere 90 Grad aufweist.

8. Vorrichtung nach einem der Ansprüche 6 bis 7,
**dadurch gekennzeichnet,**
**dass** die Petrischalenaufnahme (116) mindestens zwei, vorzugsweise vier, äquidistant angeordnete Haltefinger (117) aufweist, insbesondere wobei ein erster Haltefinger (117), insbesondere über einen Haltesteg (119), am Schwenkturm (115) angebracht ist.

9. Vorrichtung nach einem der Ansprüche 6 bis 8,
**dadurch gekennzeichnet,**
**dass** die Petrischalenaufnahme (116), insbesondere die Haltefinger (117), eine horizontal ausgerichtete Auflagefläche zur Auflage eines Bodens (122) der Petrischale (121) und eine vertikal ausgerichtete Anschlagschulter (118) zum Anschlag des Bodens (122) der Petrischale (121) aufweist.

10. Vorrichtung nach wenigstens einem der Ansprüche 1 bis 5,
**dadurch gekennzeichnet,**
**dass** die Transportvorrichtung (203) einen Transportsockel (214), eine am Transportsockel (214) gehaltene, insbesondere kreisförmig umlaufende, Führungsbahn (224) zum Führen einer Petrischale (121) vom Petrischalenspeicher (201) zum Petrischalenbetätiger (202) und einen am Transportsockel (214) schwenkbar gehaltenen Schubhebel (225) zum Bewegen der Petrischale (121) umfasst.

11. Vorrichtung nach Anspruch 10,
**dadurch gekennzeichnet,**
**dass** die Führungsbahn (224) einen Innenring (226) und einen Außenring (227) umfasst, wobei der Innenring (226) und/oder der Außenring (227) einen umlaufenden Auflagesteg (228) und/oder einen umlaufenden Anlagesteg (229) aufweist.

12. Vorrichtung nach einem der vorangehenden Ansprüche,
**dadurch gekennzeichnet,**
**dass** der Petrischalenbetätiger (102, 202) einen einen Deckel (120, 220) der Petrischale (121, 221) fixierenden Deckelgreifer (112, 212) und einen Mechanismus (113, 213) zum Anheben und/oder Verschwenken des Deckels (120, 220) umfasst.

13. Vorrichtung nach Anspruch 12,
**dadurch gekennzeichnet,**
**dass** der Deckelgreifer (120), der Schwenkturm (115) und die Petrischalenaufnahme (116) derart angeordnet sind, dass der Schwenkturm (115) beim Verschwenken der Petrischalenaufnahme (116) den Deckel (120) in den Deckelgreifer (112) überführt.

14. Verfahren zum Messen der Verkeimung eines kontrollierten, nahezu keimfreien Raumes, insbesondere eines Reinraumes, mittels einer Vorrichtung nach einem der vorherigen Ansprüche und mittels einer in einer Petrischale (121, 221) befindlichen, Keime detektierenden Nährlösung, mit den folgenden Schritten:
a.) Bereitstellen von mindestens zwei Petrischalen (121, 221) in einem im Raum befindlichen Petrischalenspeicher (101, 201) mit einer Vorrichtung (100) nach einem der vorherigen Ansprüche, wobei jede Petrischale (121, 221) mit einer Keime detektierenden Nährlösung gefüllt ist,
b.) Transportieren einer ersten Petrischale (121, 221) vom Petrischalenspeicher (101, 201) zu einem Petrischalenbetätiger (102, 202),
c.) Öffnen der ersten Petrischale (121, 221) mittels des Petrischalenbetätigers (102, 202) zu einem ersten Zeitpunkt, so dass im Raum befindliche Keime in die Nährlösung gelangen können,
d.) Schließen der ersten Petrischale (121, 221) mittels des Petrischalenbetätigers (102, 202) nach einer vorbestimmten ersten Zeitdauer, so dass keine weiteren Keime in die Nährlösung gelangen können,
e.) Rückführen der ersten Petrischale (121, 221) in den Petrischalenspeicher (101, 201),
f.) Transportieren einer weiteren Petrischale (121, 221) vom Petrischalenspeicher (101, 201) zum Petrischalenbetätiger (102, 202),
g.) Öffnen der weiteren Petrischale (121, 221) zu einem weiteren Zeitpunkt, so dass im Raum befindliche Keime in die Nährlösung gelangen können,
h.) Schließen der weiteren Petrischale (121, 221) mittels des Petrischalenbetätigers (102, 202) nach einer vorbestimmten weiteren Zeitdauer, so dass keine weiteren Keime in die Nährlösung gelangen können,
i.) Rückführen der weiteren Petrischale (121, 221) in den Petrischalenspeicher (101, 201),
j.) Abtransport der geschlossenen Petrischalen (121, 221) im Petrischalenspeicher (101, 201) aus dem Raum, insbesondere in ein Labor zur Untersuchung der Nährlösung.

15. Verfahren nach Anspruch 14,
**gekennzeichnet durch folgenden Schritt**,
k.) Wiederholen der Schritte f.) bis i.) bis alle im Petrischalenspeicher (101, 201) befindlichen Petrischalen (121, 221) im Raum befindliche Keime aufgenommen haben.

## Claims

1. Device for measuring the germ load of a controlled, virtually germ-free room, in particular a clean room, by means of a germ-detecting nutrient solution located in a Petri dish (121, 221), having a Petri dish storage (101, 201) for accommodating at least two Petri dishes (121, 221), a Petri dish actuator (102, 202) for opening and closing a Petri dish (121, 221) and a transport device (103, 203) for transporting a single Petri dish (121, 221) from the Petri dish storage (101, 201, 301) to the Petri dish actuator (102, 202) and/or for transporting a single Petri dish (121, 221) from the Petri dish actuator (102, 202) to the Petri dish storage (101, 201)
**characterized in that**
the Petri dish storage (101, 201, 301) comprises a storage base (104, 204, 304) and a lifting tower (105, 205, 305) held vertically adjustable on the storage base (104, 204, 304), wherein at least two Petri dish trays (106, 206, 306) being provided on the lifting tower (105, 205, 305).

2. Device according to claim 1,
**characterized in that**
the lifting tower (105, 205, 305) can be moved vertically by means of an adjusting device, the adjusting device being arranged in the storage base (104, 204, 304).

3. The device according to any one of claims 1 or 2,
**characterized in that**
the Petri dish trays (106, 206, 306) are removably held on the lifting tower (305).

4. Device according to at least one of claims 1 to 3,
**characterized in that**
at least two Petri dish trays (106, 206, 306) are arranged in alignment one above the other.

5. Device according to at least one of claims 1 to 4,
**characterized in that**
the Petri dish trays (106, 306) has a support ring (108) on which at least two, preferably four, inwardly oriented support tongues (109) are formed , the support tongues (109) being arranged in a lower region of the support ring (108) and thus forming a recess for receiving the Petri dish (121) and, in particular, an opening (110) oriented towards the transport device (103) being formed in the support ring (108).

6. Device according to at least one of the preceding claims,
**characterized in that**
the transport device (103) comprises a transport base (114) and a swivel tower (115) held pivotably on the transport base (114), a radially projecting Petri dish holder (116) for holding a, preferably closed, Petri dish (121) being formed on the swivel tower (115).

7. Device according to claim 6,
**characterized in that**
the swivel tower (115) is designed and arranged such that the Petri dish holder (116) can be swiveled both under the Petri dish tray (106) of the Petri dish storage (101) and under the Petri dish actuator (102), in particular **in that** the swivel tower (115) has a swivel range of 45 degrees to 360 degrees, preferably from 80 degrees to 210 degrees, in particular 90 degrees.

8. Device according to any one of claims 6 to 7,
**characterized in that**
the Petri dish holder (116) has at least two, preferably four, equidistantly arranged holding fingers (117), in particular wherein a first holding finger (117) is attached to the swivel tower (115), in particular via a holding web (119).

9. Device according to any one of claims 6 to 8,
**characterized in that**
the Petri dish holder (116), in particular the holding fingers (117), has a horizontally aligned support surface for supporting a base (122) of the Petri dish (121) and a vertically aligned stop shoulder (118) for stopping the base (122) of the Petri dish (121).

10. Device according to at least one of claims 1 to 5,
**characterized in that**
the transport device (203) comprises a transport base (214), a guide track (224) held on the transport base (214), in particular rotating in a circle, for guiding a Petri dish (121) from the Petri dish storage (201) to the Petri dish actuator (202) and a push lever (225) pivotably held on the transport base (214) for moving the Petri dish (121).

11. Device according to claim 10,
**characterized in that**
the guide track (224) comprises an inner ring (226) and an outer ring (227), the inner ring (226) and/or the outer ring (227) having a circumferential support web (228) and/or a circumferential abutment web (229).

12. Device according to one of the preceding claims,
**characterized in that**
the Petri dish actuator (102, 202) comprises a lid gripper (112, 212) fixing a lid (120, 220) of the Petri dish (121, 221) and a mechanism (113, 213) for lifting and/or pivoting the lid (120, 220).

13. Device according to claim 12,
**characterized in that**
the lid gripper (112), the swivel tower (115) and the Petri dish holder (116) are arranged in such a way that the swivel tower (115) transfers the lid (120) into the lid gripper (112) when the Petri dish holder (116) is swiveled.

14. Method for measuring the microbial contamination of a controlled, virtually germ-free room, in particular a clean room, by means of a device according to one of the preceding claims and by means of a germ-detecting nutrient solution located in a Petri dish (121, 221), comprising the following steps:
a.) Providing at least two Petri dishes (121, 221) in a Petri dish storage (101, 201) located in the room with a device (100) according to one of the previous claims, wherein each Petri dish (121, 221) is filled with a germ-detecting nutrient solution,
b.) Transporting a first Petri dish (121, 221) from the Petri dish storage (101, 201) to a Petri dish actuator (102, 202),
c.) Opening the first Petri dish (121, 221) by means of the Petri dish actuator (102, 202) at a first point in time, so that germs in the room can enter the nutrient solution,
d.) Closing the first Petri dish (121, 221) by means of the Petri dish actuator (102, 202) after a predetermined first period of time, so that no further germs can enter the nutrient solution,
e.) Return the first Petri dish (121, 221) to the Petri dish storage (101, 201),
f.) Transporting a further Petri dish (121, 221) from the Petri dish storage (101, 201) to the Petri dish actuator (102, 202),
g.) Open the other Petri dish (121, 221) at another time so that germs in the room can enter the nutrient solution,
h.) Closing the further Petri dish (121, 221) by means of the Petri dish actuator (102, 202) after a predetermined further period of time, so that no further germs can enter the nutrient solution,
i.) Returning the other Petri dish (121, 221) to the Petri dish storage (101, 201),
j.) Removal of the closed Petri dishes (121, 221) in the Petri dish storage (101, 201) from the room, in particular to a laboratory for examination of the nutrient solution.

15. Method according to claim 14,
**characterized by** the following step:
k.) Repeat steps f.) to i.) until all Petri dishes (121, 221) in the Petri dish storage (101, 201) have taken up germs in the room.

## Revendications

1. Dispositif pour mesurer la contamination d'un espace contrôlé, pratiquement exempt de germes, en particulier d'une salle blanche, au moyen d'une solution nutritive détectant les germes et se trouvant dans une boîte de Pétri (121, 221), avec un réservoir de boîtes de Pétri (101, 201) pour recevoir au moins deux boîtes de Pétri (121, 221), avec un actionneur de boîtes de Pétri (102, 202) pour ouvrir et fermer une boîte de Pétri (121, 221) et avec un dispositif de transport (103, 203) pour transporter une seule boîte de Pétri (121, 221) du réservoir de boîtes de Pétri (101, 201, 301) à l'actionneur de boîtes de Pétri (102, 202) et/ou pour transporter une seule boîte de Pétri (121, 221) de l'actionneur de boîtes de Pétri (102, 202) au réservoir de boîtes de Pétri (101, 201)
**caractérisé en ce que**
le réservoir de boîtes de Petri (101, 201, 301) comprend un socle de stockage (104, 204, 304) et une tour de levage (105, 205, 305) maintenue de manière réglable verticalement sur le socle de stockage (104, 204, 304), au moins deux supports de boîtes de Petri (106, 206, 306) étant prévus sur la tour de levage (105, 205, 305).

2. Dispositif selon la revendication 1,
**caractérisé en ce que**
la tour de levage (105, 205, 305) peut être déplacée verticalement au moyen d'un dispositif de réglage, le dispositif de réglage étant disposé dans le socle de stockage (104, 205, 305).

3. Dispositif selon l'une des revendications 1 ou 2,
**caractérisé en ce que**
les plateaux de boîtes de Pétri (306) sont maintenus de manière amovible sur la tour de levage (305).

4. Dispositif selon au moins l'une des revendications 1 à 3,
**caractérisé en ce**
**qu'**au moins deux plateaux de boîtes de Pétri (106, 206, 306) sont disposés en alignement l'un au-dessus de l'autre.

5. Dispositif selon au moins l'une des revendications 1 à 4,
**caractérisé en ce que**
le support de boîte de Pétri (106, 306) présente un anneau de support (108) sur lequel sont formées au moins deux, de préférence quatre, languettes de support (109) orientées vers l'intérieur , les languettes de support (109) étant disposées dans une zone inférieure de l'anneau de support (108) et formant ainsi un renfoncement pour recevoir la boîte de Pétri (121) et en particulier une ouverture (110) orientée vers le dispositif de transport (103) étant formée dans l'anneau de support (108).

6. Dispositif selon au moins l'une des revendications précédentes,
**caractérisé en ce que**
le dispositif de transport (103) comprend un socle de transport (114) et une tour pivotante (115) maintenue de manière pivotante sur le socle de transport (114), un logement de boîte de Pétri (116) faisant saillie radialement étant réalisé sur la tour pivotante (115) pour recevoir une boîte de Pétri (121), de préférence fermée.

7. Dispositif selon la revendication 6,
**caractérisé en ce que**
la tour pivotante (115) est conçue et disposée de manière à pouvoir pivoter de telle sorte que le logement de boîte de Pétri (116) puisse pivoter aussi bien sous le support de boîte de Pétri (106) du réservoir de boîte de Pétri (101) que sous l'actionneur de boîte de Pétri (102), en particulier **en ce que** la tour pivotante (115) présente une plage de pivotement de 45 degrés à 360 degrés, de préférence de 80 degrés à 210 degrés, en particulier de 90 degrés.

8. Dispositif selon l'une quelconque des revendications 6 à 7,
**caractérisé en ce que**
le logement de boîte de Pétri (116) présente au moins deux, de préférence quatre, doigts de maintien (117) disposés de manière équidistante, en particulier un premier doigt de maintien (117) étant monté sur la tour pivotante (115), en particulier par l'intermédiaire d'une barre de maintien (119).

9. Dispositif selon l'une quelconque des revendications 6 à 8,
**caractérisé en ce que**
le logement de boîte de Pétri (116), en particulier les doigts de maintien (117), présente une surface d'appui orientée horizontalement pour l'appui d'un fond (122) de la boîte de Pétri (121) et un épaulement de butée (118) orienté verticalement pour la butée du fond (122) de la boîte de Pétri (121).

10. Dispositif selon au moins l'une des revendications 1 à 5,
**caractérisé en ce que**
le dispositif de transport (203) comprend un socle de transport (214), une glissière de guidage (224) maintenue sur le socle de transport (214), en particulier circulaire, pour guider une boîte de Pétri (121) du réservoir de boîte de Pétri (201) à l'actionneur de boîte de Pétri (202) et un levier de poussée (225) maintenu de manière pivotante sur le socle de transport (214) pour déplacer la boîte de Pétri (121).

11. Dispositif selon la revendication 10,
**caractérisé en ce que**
la glissière de guidage (224) comprend une bague intérieure (226) et une bague extérieure (227), la bague intérieure (226) et/ou la bague extérieure (227) présentant une nervure d'appui (228) périphérique et/ou une nervure d'appui (229) périphérique.

12. Dispositif selon l'une des revendications précédentes,
**caractérisé en ce que**
l'actionneur de boîte de Pétri (102, 202) comprend un dispositif de préhension de couvercle (112, 212) fixant un couvercle (120, 220) de la boîte de Pétri (121, 221) et un mécanisme (113, 213) pour soulever et/ou faire pivoter le couvercle (120, 220).

13. Dispositif selon la revendication 12,
**caractérisé en ce que**
le dispositif de préhension de couvercle (112), la tour pivotante (115) et le logement de boîte de Pétri (116) sont disposés de telle sorte que la tour pivotante (115) transfère le couvercle (120) dans le dispositif de préhension de couvercle (112) lors du pivotement du logement de boîte de Pétri (116).

14. Procédé de mesure de la contamination par des germes d'un espace contrôlé pratiquement exempt de germes, en particulier d'une salle blanche, au moyen d'un dispositif selon l'une des revendications précédentes et au moyen d'une solution nutritive détectant des germes et se trouvant dans une boîte de Pétri (121, 221), comprenant les étapes suivantes:
a.) Mise à disposition d'au moins deux boîtes de Pétri (121, 221) dans un réservoir de boîtes de Pétri (101, 201) situé dans l'espace avec un dispositif (100) selon l'une des revendications précédentes, dans lequel chaque boîte de Pétri (121, 221) est remplie d'une solution nutritive détectant les germes,
b.) Transporter une première boîte de Pétri (121, 221) depuis le stockage de boîtes de Pétri (101, 201) vers un actionneur de boîtes de Pétri (102, 202),
c.) Ouverture de la première boîte de Pétri (121, 221) au moyen de l'actionneur de boîte de Pétri (102, 202) à un premier moment, de sorte que les germes se trouvant dans l'espace peuvent passer dans la solution nutritive,
d.) Fermeture de la première boîte de Pétri (121, 221) au moyen de l'actionneur de boîte de Pétri (102, 202) après une première période de temps prédéterminée, de sorte qu'aucun autre germe ne puisse pénétrer dans la solution nutritive,
e.) Le retour de la première boîte de Pétri (121, 221) dans le réservoir de boîtes de Pétri (101, 201),
f.) Transporter une autre boîte de Pétri (121, 221) depuis le réservoir de boîtes de Pétri (101, 201) jusqu'à l'actionneur de boîte de Pétri (102, 202),
g.) Ouverture de l'autre boîte de Pétri (121, 221) à un autre moment, de sorte que les germes présents dans la pièce puissent passer dans la solution nutritive,
h.) Fermeture de l'autre boîte de Pétri (121, 221) au moyen de l'actionneur de boîte de Pétri (102, 202) après une autre période de temps prédéterminée, de sorte qu'aucun autre germe ne puisse pénétrer dans la solution nutritive,
i.) Retour de l'autre boîte de Pétri (121, 221) dans le réservoir de boîtes de Pétri (101, 201),
j.) Evacuation des boîtes de Pétri fermées (121, 221) dans le réservoir de boîtes de Pétri (101, 201) hors de la pièce, en particulier vers un laboratoire pour l'analyse de la solution nutritive.

15. Procédé selon la revendication 14,
**caractérisé par** l'étape suivante
k.) Répéter les étapes f.) à i.) jusqu'à ce que toutes les boîtes de Pétri (121, 221) se trouvant dans le réservoir de boîtes de Pétri (101, 201) aient absorbé des germes présents dans la chambre.
